# EUROPEAN PATENT APPLICATION

(11) **EP 4 640 146 A1**
(43) Date of publication of application: **29.10.2025**
(21) Application number: 24172466.5
(22) Date of filing: 25.04.2024
(51) Int. Cl.: A61B 5/022

(54) **BLOOD PRESSURE CUFF AND BLOOD PRESSURE DETERMINATION SYSTEM COMPRISING THE BLOOD PRESSURE CUFF**

(71) Applicant: Koninklijke Philips N.V., 5656 AG Eindhoven (NL)
(72) Inventor: PFEIFFER, Ulrich, Eindhoven (NL); FAEHLE, Thomas Matthias, Eindhoven (NL); HOFFMANN, Benjamin, 5656AG Eindhoven (NL); FRANK, Katrin, Eindhoven (NL)
(74) Representative: Philips Intellectual Property & Standards

(57) **Abstract**

The invention relates to a blood pressure cuff 4 configured to surround a body part 11 of a subject for determining the blood pressure of the subject, wherein the blood pressure cuff comprises an inflatable bladder 13 with an inner layer and an outer layer, wherein a) the blood pressure cuff further comprises a shell 12 having an inner side merged with the outer layer of the bladder, and/or b) the outer layer of the bladder is more rigid than the inner layer of the bladder. By using such a blood pressure cuff, the quality of measured pressure pulses indicative of the pressure within the bladder, can be increased significantly, which allows for blood pressure measurements having an increased accuracy.

## Description

### FIELD OF THE INVENTION

The invention relates to a blood pressure cuff, a blood pressure determination system comprising the blood pressure cuff and a blood pressure determination method. The invention relates further to a set of blood pressure cuffs and to the use of the set of blood pressure cuffs.

### BACKGROUND OF THE INVENTION

A known blood pressure determination system for determining the blood pressure of a subject is, for instance, an oscillometric system. An oscillometric system comprises a blood pressure cuff with a bladder that is connected to an air pump and to a valve system for inflating and deflating the bladder. During inflation and/or deflation, pressure pulses, which are initially caused by pulse waves within the brachial artery of the subject, are measured by using a pressure sensor, wherein the blood pressure of the subject is determined depending on the measured pressure pulses. The blood pressure determined by such an oscillometric system might not be accurate enough.

### SUMMARY OF THE INVENTION

It is an object of the present invention to provide a blood pressure cuff and a blood pressure determination system comprising the blood pressure cuff which allow for a more accurate determination of the blood pressure. It is a further object of the present invention to provide a set of these blood pressure cuffs and to provide a specific use of this set of blood pressure cuffs. It is also an object of the present invention to provide a blood pressure determination method.

In a first aspect of the invention a blood pressure cuff configured to surround a body part of a subject for determining the blood pressure of the subject is presented, wherein the blood pressure cuff comprises an inflatable bladder with an inner layer and an outer layer, wherein a) the blood pressure cuff further comprises a shell having an inner side merged with the outer layer of the bladder, and/or b) the outer layer of the bladder is more rigid than the inner layer of the bladder.

It has been found that, if the outer layer of the bladder and the inner side of the shell are merged and/or if the outer layer of the bladder is more rigid than the inner layer of the bladder, the quality of pressure pulses, which can be measured by a pressure sensor of a blood pressure determination system comprising the blood pressure cuff and which are indicative of the pressure within the bladder, can be increased significantly. For instance, the amplitude of these pressure pulses can be increased and/or the contours of the pressure pulses can be identified more accurately. Determining the blood pressure of the subject based on the pressure pulses, which have a higher quality, increases the accuracy of the blood pressure determination.

The shell preferentially is conical. The conical shell can be better adapted to the shape of the surrounded body part, which can lead to a further increased accuracy of the blood pressure determination.

It is noted that the expression "A and/or B" includes i) A without B, ii) B without A and iii) A and B. For instance, the expression "wherein a) the blood pressure cuff further comprises a shell having an inner side merged with the outer layer of the bladder, and/or b) the outer layer of the bladder is more rigid than the inner layer of the bladder" includes that i) the blood pressure cuff comprises a shell having an inner side merged with the outer layer of the bladder, without the outer layer of the bladder being more rigid than the inner layer of the bladder, ii) the outer layer of the bladder is more rigid than the inner layer of the bladder, without the blood pressure cuff further comprising a shell having an inner side merged with the outer layer of the bladder, and iii) the blood pressure cuff further comprises a shell having an inner side merged with the outer layer of the bladder and the outer layer of the bladder is more rigid than the inner layer of the bladder. It is further noted that the expression "A and/or B" does not exclude that further features are present, for instance a feature "C". For example, in the case of option ii) above, also a shell can be present, of which the inner side is not merged with the outer layer of the bladder. However, preferentially, in the case of option ii) the blood pressure cuff does not comprise any shell.

The bladder and also the shell, if present, are configured to surround the body part. In particular, the outer layer of the bladder and the inner side of the shell can be merged and then wrapped around the body part. The outer layer of the bladder and the inner side of the shell can be merged, for instance, by gluing or welding them together. However, they can also be merged by using another merging technique.

The merging can also be regarded as being an attaching. Preferentially, at least 80%, further preferred more than 90% and even further preferred more 95% of the facing outer layer of the bladder and the facing inner side of the shell, i.e., of the outer layer and the inner side which face each other, are merged. In the most preferred embodiment, 100% of the outer layer of the bladder and the inner side of the shell, which face each other, are merged.

In an example, an outer side of the outer layer of the bladder, which is merged with the inner side of the shell, is sleek. If the outer side of the outer layer of the bladder is sleek, there are less likely air bubbles or the like between this outer side of the outer layer of the bladder and the merged inner side of the shell. This can allow for a further increased quality of the pressure pulses and correspondingly for an even more accurate blood pressure determination.

Moreover, in an example the shell comprises at least one of high density polyethylene (HDPE), polypropylene (PP) and acrylonitrile butadiene styrene (ABS). In a preferred embodiment, the shell comprises a blend of HDPE, PP and ABS. It is even more preferred that the shell consists of a HDPE, PP, ABS blend or a material with similar physical properties which can preferably be injection molded. It has been found that, by using this shell material, the quality of the pressure pulses can be even further increased, which allows for a further increased accuracy of the blood pressure determination. Furthermore, these materials are biocompatible and are either biodegradable or can be recycled.

In an example, an inner layer of the bladder and the outer layer of the bladder comprise different materials. This allows to independently adapt the inner layer and the outer layer of the bladder to different functions. For instance, the material of the inner layer of the bladder can be chosen such that it is non-adhesive on the subject's skin. The outer layer of the bladder can comprise a material or be made of a material that allows for a high-quality merge with the inner side of the shell.

The outer layer of the bladder can comprise a material being thicker and/or more rigid and/or harder than the material comprised by the inner layer of the bladder. Thus, the outer layer of the bladder can be made of a material being thicker and/or more rigid and/or harder than the material of the inner layer of the bladder. The outer layer of the bladder can comprise a reinforcing structure, whereas the inner layer preferentially does not comprise this reinforcing structure.

In an example, the outer layer of the bladder can comprise or be made of a ripstop material like a ripstop nylon or a ripstop polyester. The material of the outer layer can also be a combination of a ripstop nylon and a ripstop polyester. The inner layer of the bladder can comprise or can be made of a thermoplastic polyurethane (TPU) that preferentially is non-adhesive on the subject's skin. Instead, or in addition to thermoplastic TPU, also another material can be used, which preferentially has similar physical properties. For instance, a phthalate-free polyvinyl chloride (PVC) might be used.

However, it is also possible that the inner layer of the bladder and the outer layer of the bladder comprise the same material. In particular, in an embodiment, they are made of the same material. This can be the case if the outer layer of the bladder is merged with the inner side of this shell.

Preferentially, the ratio of the area of the outer layer relative to the area of the inner layer of the bladder is larger than one. Moreover, the inner layer of the bladder can be shorter than the outer layer of the bladder. This can also lead to a further increased quality of the pressure pulses and hence a further increased accuracy of the blood pressure that is determined based on the pressure pulses. These comparisons preferentially refer to the parts of the inner layer and the outer layer, which enclose the fluid, if pumped into the bladder. These parts hence do not include, for instance, flares and welding seams. Thus, the ratio preferentially considers the parts of the outer and inner layers which are adjacent the fluid, when the fluid has been pumped into the bladder. Also, the shortness criterion preferentially refers to these parts of the inner and outer layers. However, the ratio criterion and/or the shortness criterion can also refer to the entire inner layer and the entire outer layer and not just to the parts of these layers which enclose the fluid when it has been pumped into the bladder.

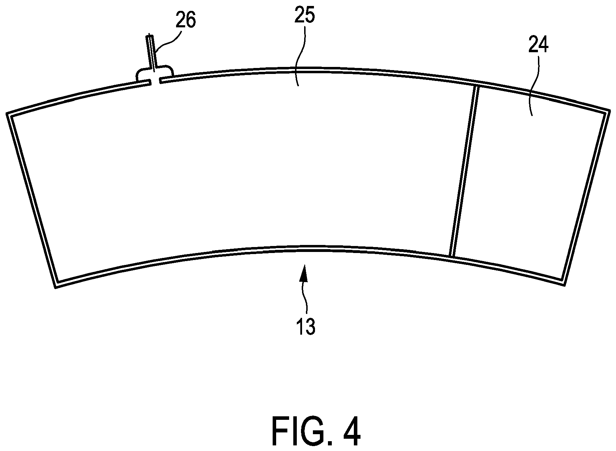

The shortness criterion preferentially refers to the length direction of the bladder being the direction along the longest dimension when the bladder is unwrapped as exemplarily illustrated in Fig. 4. This length direction follows the shape of the unwrapped bladder and therefore can be slightly curved as also exemplarily illustrated in Fig. 4.

In an example, the inner layer of the bladder is thinner than the outer layer of the bladder. A relatively thin inner layer can improve the transfer of the pulse waves from the artery through the tissue into the bladder, thereby allowing for a further improved quality of the pressure pulses, wherein this, in turn, can further increase the accuracy of the determined blood pressure.

In an example, the blood pressure cuff comprises a flap with an attachment means for attaching the flap to the outer side of the blood pressure cuff, in order to thereby fasten the blood pressure cuff when it surrounds the subject's body part, wherein the flap comprises at least one of the outer layer and the inner layer of the bladder. It is preferred that the flap is formed by merging parts of the inner and outer layers of the bladder. It is also preferred that the flap is formed as an end section of the bladder. This allows for a simplified manufacturing of the blood pressure cuff because it is not required to use necessarily additional material for securing the blood pressure cuff on the body part of the subject, while the blood pressure cuff surrounds the body part.

Preferentially, the dimensions of the shell are such that the shell covers a part of the bladder, in which fluid is flowable, completely. In particular, preferentially the shell completely surrounds the body part. This allows for a further improved quality of the pressure pulses that are used for determining the blood pressure, wherein this leads to a further increased accuracy of the determined blood pressure.

In a further aspect of the present invention a set of the blood pressure cuffs is presented, wherein the set includes blood pressure cuffs with shells having different cone angles. Preferentially, the set comprises a first blood pressure cuff for subjects having a subject length, which is a length of a predefined part of the body of the subject or a length of the entire body of the subject, being larger than a predefined length threshold and a second blood pressure cuff for subjects having a subject length being smaller than the predefined length threshold, wherein the cone angle of the first blood pressure cuff is smaller than the cone angle of the second blood pressure cuff. The subjects, to which it is referred here, are preferentially obese subjects. Obese subjects are preferentially defined as being subjects having a body mass index being larger than a predefined body mass index threshold of, for instance, 30 kg/m².

If the subject length refers to a predefined part of the body of the subject, the predefined part preferentially is the body part, which is surrounded by the respective blood pressure cuff while determining the blood pressure, for instance, the upper arm of the subject. If the subject length refers to the length of the entire body, the subject length could also be regarded as being the body height. In this case the length threshold might be 165 cm. However, the length threshold can also have another value.

In another aspect of the present invention a use of the set of the blood pressure cuffs for determining the blood pressure of subjects having different subject lengths is presented, wherein, for determining the blood pressure for subjects having a subject length being larger than the predefined length threshold, the first blood pressure cuff is used and, for determining the blood pressure for subjects having a subject length being smaller than the predefined length threshold, the second blood pressure cuff is used. It has been found that, if such a set of blood pressure cuffs is provided and used for determining the blood pressure of obese subjects of different subject lengths, the quality of the measured pressure pulses can be even further improved for subjects. This can finally result in a more accurate blood pressure determination for subjects. Also, this preferentially refers to obese subjects.

In a further aspect of the present invention a blood pressure determination system for determining the blood pressure of a subject is presented, wherein the blood pressure determination system comprises a) a blood pressure cuff as defined by any of claims 1 to 11, b) an inflation device configured to inflate and deflate the bladder of the blood pressure cuff, c) a pressure measuring device being configured to measure the pressure in the bladder during inflation and/or deflation and d) a blood pressure determination device for determining the blood pressure based on the measured pressure in the bladder. In particular, the pressure measuring device is configured to measure a sequence of pressure pulses during inflation and/or deflation and the blood pressure determination device is configured to determine the blood pressure based on the measured sequence of pressure pulses. For determining the blood pressure based on the measured sequence of pressure pulses, known oscillometric algorithms or other algorithms can be used.

In another aspect of the present invention a blood pressure determination method for determining the blood pressure of a subject is presented, wherein the blood pressure determination method comprises:
- wrapping a blood pressure cuff as defined by any of claims 1 to 11 around a body part of a subject,
- inflating and deflating the bladder of the blood pressure cuff by an inflation device,
- measuring the pressure in the bladder by a pressure measuring device during inflation and/or deflation,
- determining the blood pressure based on the measured pressure in the bladder by a blood pressure determination device.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of a blood pressure determination system for determining the blood pressure of a subject,
Fig. 2 shows schematically and exemplarily a perspective view of a blood pressure cuff of the blood pressure determination system,
Fig. 3 shows schematically and exemplarily a sectional view of the blood pressure cuff at minimal circumference,
Fig. 4 shows schematically and exemplarily a bladder of the blood pressure cuff,
Fig. 5 shows schematically and exemplarily a sectional view of the blood pressure cuff before closing the blood pressure cuff around an upper arm of the subject,
Fig. 6 shows schematically and exemplarily a sectional view of the blood pressure cuff at maximal circumference,
Fig. 7 shows a flowchart exemplarily illustrating a blood pressure determination method for determining the blood pressure of a subject,
Fig. 8 illustrates exemplarily a decreased bladder volume,
Figs. 9 and 10 show a comparison between pressure pulses measured by different blood pressure cuffs,
Fig. 11 shows a comparison of pressure pulses, which have been measured by using different blood pressure cuffs, at diastolic arterial pressure (DAP),
Fig. 12 shows a comparison of pressure pulses, which have been measured by using different blood pressure cuffs, at MAP,
Fig. 13 shows a comparison of pressure pulses, which have been measured by using different blood pressure cuffs, at systolic arterial pressure (SAP),
Fig. 14 shows a further comparison of pressure pulses, which have been measured by using different blood pressure cuffs, at DAP,
Fig. 15 shows a further comparison of pressure pulses, which have been measured by using different blood pressure cuffs, at MAP,
Fig. 16 shows a further comparison of pressure pulses, which have been measured by using different blood pressure cuffs, at SAP, and
Fig. 17 shows schematically and exemplarily a sectional view of a further blood pressure cuff.

### DETAILED DESCRIPTION OF EMBODIMENTS

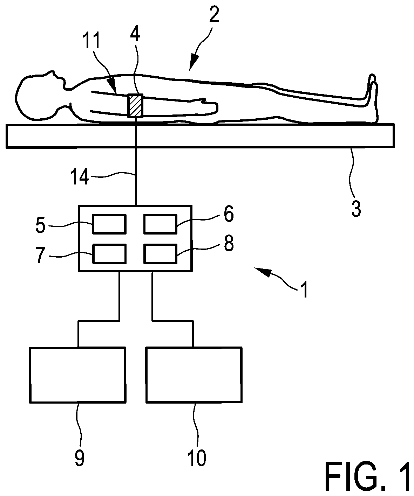

Fig. 1 shows schematically and exemplarily an embodiment of a blood pressure determination system for determining the blood pressure of a subject. The blood pressure determination system 1 comprises a blood pressure cuff 4 wrapped around an upper arm 11 of a subject 2 who is located on a support means 3 like a patient table or bed. The blood pressure cuff 4 comprises a bladder which will be explained in more detail further below and which is connected via a tube 14 with an inflation device 5 that is configured to inflate and deflate the bladder of the blood pressure cuff 4.

The inflation device 5 preferentially comprises a pump and a valve system, wherein the bladder is inflated by pumping a fluid, preferentially air, into the bladder and the bladder is deflated by controlling the valve system to open the inside of the bladder via the tube 14 and the inflation device 5 to the surrounding outside of the blood pressure determination system 1.

The blood pressure determination system 1 further comprises a pressure measuring device 6 being configured to measure the pressure in the bladder of the blood pressure cuff 4 during inflation and/or deflation. In particular, pressure pulses caused within the bladder of the blood pressure cuff 4 by pulse waves within the brachial artery are transmitted to the pressure measuring device 6 via the tube 14, wherein the pressure measuring device 6 comprises a pressure sensor for measuring the transmitted pressure pulses, i.e. the transmitted sequence of pressure pulses. The measured pressure pulses are provided to a blood pressure determination device 7 that is configured to determine the blood pressure based on the measured pressure pulses. The inflation device 5, the pressure measuring device 6 and the blood pressure determination device 7 can be controlled by a controller 8.

The inflation device 5, the pressure measuring device 6 and the blood pressure determination device 7 can be known devices generally used for inflating and deflating a blood pressure cuff, for measuring pressure pulses and for determining blood pressure based on the measured pressure pulses by using an oscillometric algorithm. The blood pressure determination system 1 further comprises an input device 9 like a keyboard, a touchpad, et cetera and an output device 10 like a display.

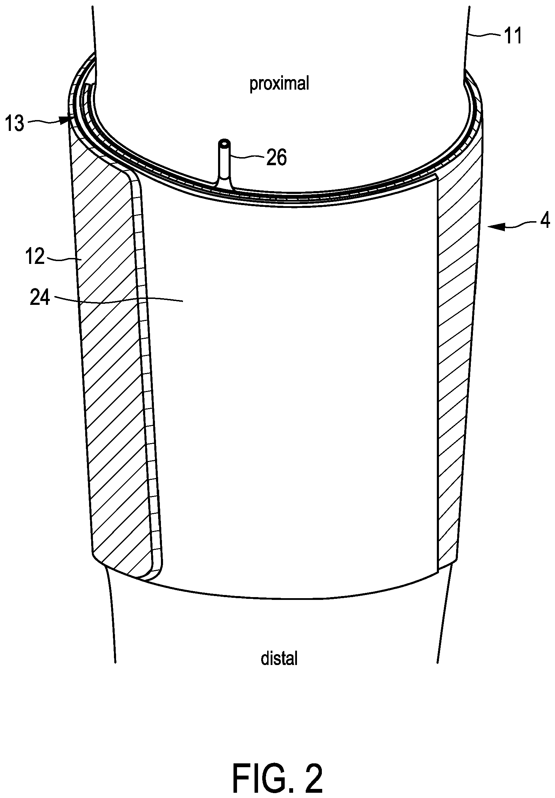

Fig. 2 shows schematically and exemplarily an embodiment of the blood pressure cuff 4 in more detail.

The blood pressure cuff 4 comprises the inflatable bladder 13 and a conical shell 12, wherein the bladder 13 and the shell 12 are configured to be wrapped around the upper arm 11 of the subject 2 such that the bladder 13 and the shell 12 surround the upper arm 11. A tube 26, which is fluidly connected to the inside of the bladder 13, can be used for pumping fluid, especially air, into the bladder 13 and also for letting the fluid out of the bladder 13. The tube 14, which is schematically and exemplarily illustrated in Fig. 1, can be connected to the smaller tube 26, in order to allow the inflation device 5 to inflate and deflate the bladder 13 of the blood pressure cuff 4 via the tubes 14 and 26.

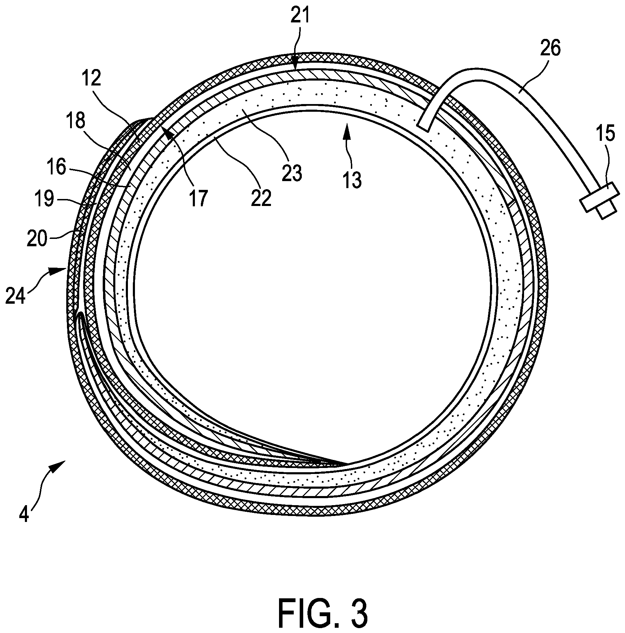

Fig. 3 shows schematically and exemplarily a sectional view through the blood pressure cuff 4. As can be seen in Fig. 3, the bladder 13 comprises an outer layer 16 and an inner layer 22, wherein these two layers 16 and 22 are attached to each other, for instance, glued or welded, along their edges, in order to form in between these two layers 16, 22 a space 23 that can be filled with a fluid like air by using the tube 26 which comprises a connector 15 for connecting the tube 26 to the tube 14.

The bladder 13 is also schematically and exemplarily illustrated in Fig. 4. As can be seen in Fig. 4, the space of the bladder 13, in which the fluid can be pumped, is within the main part 25 of the bladder 13, wherein the bladder 13 also comprises a smaller end section 24 in which no fluid can be pumped and which forms a flap 24. In the end section 24 no fluid can be pumped, because in this end section the inner and outer layers of the bladder 13 are completely merged to each other, for instance, by welding or gluing. The end section or flap 24 comprises attachment means for attaching the flap 24 to the outer side of the shell 12. By attaching the flap 24 to the outer side of the shell 12, the blood pressure cuff 4 can be fastened when it surrounds the subject's upper arm 11.

The flap 24 comprises as the attachment means a loop part 20 that attaches to a hook part 19 on the outer side of the shell 12. The loop part 20 of the flap 24 and the hook part 19 on the outer side of the shell 12 are arranged such that the blood pressure cuff 4 can be fastened when it surrounds the subject's body part 11.

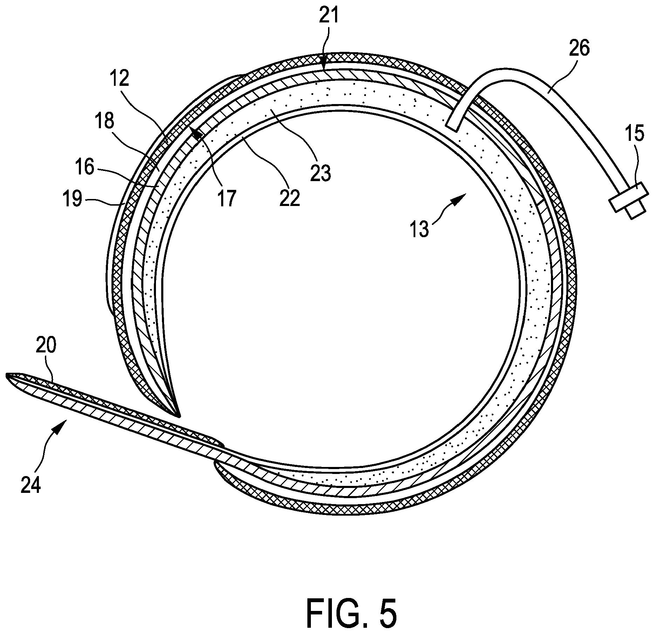

In Fig. 3 the flap 24 has been attached to the outer side of the shell 12, whereas in Fig. 5 the flap 24 is shown before it is attached to the outer side of the shell 12 or after it has been released from the outer side of the shell 12.

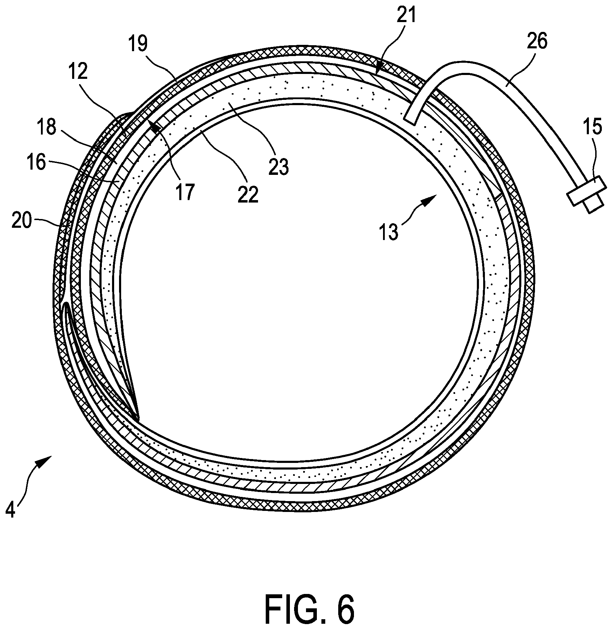

The diameter of the blood pressure cuff 4 depends on the position of the flap 24 on the outer side of the shell 12. Fig. 3 illustrates the blood pressure cuff 4 in a situation in which the diameter is relatively small, and Fig. 6 illustrates the same blood pressure cuff 4 in a situation in which the diameter is relatively large. In particular, in Fig. 3 the minimal circumference of the blood pressure cuff 4 is shown and in Fig. 6 the maximal circumference of the blood pressure cuff 4 is shown. Thus, the same size blood pressure cuff preferentially has an adaptable circumference and hence diameter such that the same blood pressure cuff can be used for different subjects having different diameters of the upper arm, wherein the respective blood pressure cuff can comprise a working range having a lower limit that defines the minimal circumference and an upper limit that defines the maximal circumference.

It should be noted that the Figures, particularly Figs. 3, 5 and 6, are not to scale and do not represent real size relations. For instance, in Figs. 3 and 6 the diameter of the blood pressure cuff seems to be the same, although they are different. The Figures are just for illustrative purposes and schematic. Moreover, they illustrate a situation in which they are pressurized, i.e. in which the bladder contains the fluid which preferentially is air, wherein, although not shown in the schematic Figures, the inner layer may comprise one or very few and very small folds.

The shell 12 comprises an inner side 17, wherein the outer layer 16 of the bladder 13, i.e. the outer side 21 of the outer layer 16 of the bladder 13, and the inner side 17 of the shell 12 are merged and wherein in this embodiment this merging means that they are glued together or welded together. In Fig. 3 exemplarily a glue is indicated by reference number 18. The outer side 21 of the outer layer 16 of the bladder 13 is sleek such that the gluing or welding is possible without any air inclusions in between the outer side 21 of the outer layer 16 and the inner side 17 of the shell 12.

The terms "outer layer of the bladder", "inner layer of the bladder" and "inner side of the shell" refer to the blood pressure cuff surrounding the body part, i.e., for instance, the inner layer of the bladder is directed towards the surrounded body part and the outer layer of the bladder is directed away from the surrounded body part. Moreover, the inner side of the shell is directed towards the surrounded body part.

The shell 12 is more rigid, though flexible, than the bladder 13 and it is made of HDPE, PP, ABS blend or a material with similar physical properties which can preferably be injection molded. The inner layer 22 of the bladder 13 and the outer layer 16 of the bladder 13 are made of different materials. The outer layer 16 of the bladder 13 is made of a ripstop material, wherein the ripstop material is a ripstop nylon, a ripstop polyester or a combination of the ripstop nylon and the ripstop polyester. For the inner layer 22 of the bladder 13 a material is used, which is non-adhesive on the subject's skin. In a preferred embodiment, the inner layer 22 of the bladder 13 is made of TPU or materials with similar physical properties, e.g. phthalate-free PVC. The material used for the inner layer preferentially is airtight, biocompatible, resistant to disinfectants and dimensionally stable.

The ratio of the area of the outer layer 16 of the bladder 13 relative to the area of the inner layer 22 of the bladder 13 is larger than one. This is in contrast to known blood pressure cuffs in which this ratio generally is one, since 2D-welded. Moreover, the inner layer 22 of the bladder 13 is shorter and thinner than the outer layer 16 of the bladder 13. The shell 12 is larger than and completely covers the main part 25 of the bladder 13, in which the fluid can be pumped. Furthermore, the blood pressure cuff 4 is constructed such that the shell 12 completely surrounds the body part 11, after the blood pressure cuff 4 has been wrapped around the upper arm 11.

A set of blood pressure cuffs can be provided, wherein the set includes blood pressure cuffs with shells having different cone angles. In particular, the set can comprise a first blood pressure cuff for obese subjects having a subject length being larger than a predefined length threshold and a second blood pressure cuff for obese subjects having a subject length being smaller than the predefined length threshold, wherein the cone angle of the first blood pressure cuff is smaller than the cone angle of the second blood pressure cuff. The subject length can be, for instance, the length of the body part, which is to be surrounded by the blood pressure cuff, or the body height. The predefined length threshold can be, for instance, 165 cm, if the subject length is the body height.

This set of blood pressure cuffs can be used for determining the blood pressure of obese subjects of different subject lengths. For determining the blood pressure for obese subjects having a subject length being larger than the predefined length threshold, the first blood pressure cuff is used and, for determining the blood pressure for obese subjects having a subject length being smaller than the predefined length threshold, the second blood pressure cuff is used.

In particular, the set of blood pressure cuffs can comprise, for adults, at least four different cone angles for four different classes that could be named small adult SA, adult A, large adult LA and large adult short LAS, wherein the cone angles increase from the class small adult SA to the class large adult short LAS. The class large adult short LAS includes obese subjects having a subject length being smaller than the length threshold and the class large adult LA includes obese subjects having a subject length being larger than the length threshold. A subject is regarded as being obese if its body mass index (BMI) is larger than a predefined BMIthreshold of, for instance, 30 kg/m².

Thus, the conus angles of the blood pressure cuffs for the classes LA and LAS differ and are inversely related to the subject length, i.e., for instance, shorter obese subjects have a higher conus angle, whilst the conus angle is smaller in taller obese subjects.

The set of blood pressure cuffs can also comprise blood pressure cuffs for adolescents, children and infants, wherein different classes can be defined depending on the upper arm length, the medial upper arm circumference and whether the subject is obese or not and wherein for the different classes blood pressure cuffs comprising different cone angles can be provided from strongly conical to almost cylindrical.

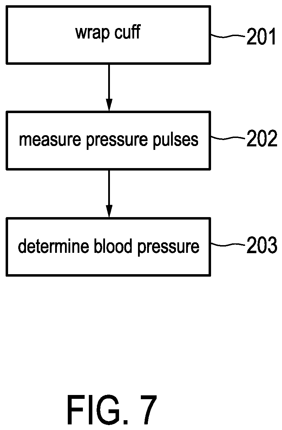

In the following an embodiment of a blood pressure determination method will exemplarily be described with reference to a flow chart shown in Fig. 7.

In step 201, the blood pressure cuff is wrapped around the upper arm of the subject. In step 202, the bladder of the blood pressure cuff is inflated and deflated and the pressure in the bladder is measured during inflation and/or deflation. In step 203, the blood pressure is determined based on the measured pressure in the bladder. Thus, a sequence of pressure pulses is measured by using the pressure measuring device 6, while the bladder is inflated and/or deflated by using the inflation device 5, wherein the blood pressure determination device 7 determines the blood pressure based on the measured sequence of pressure pulses. For determining the blood pressure based on the measured sequence of pressure pulses known oscillometric algorithms can be used.

Known blood pressure cuffs are generally cylindrical when they have been wrapped around a subject's upper arm. However, more than 95% of upper arms of adults are conical. For this reason, in contrast to the known blood pressure cuffs, the blood pressure cuff described above has a conical shell, thereby better fitting to the upper arms of adults. This contributes to the improved quality of the measured pressure pulses.

Moreover, known blood pressure cuffs tend to balloon in inflation, which can result in a loss of signal to the atmosphere on the outer side of the blood pressure cuff. The blood pressure cuff described above avoids ballooning in inflation, exhibits a lower air volume/pressure slope and thereby prevents the signal loss to the atmosphere. Also, this contributes to the improved quality of the measured pressure pulses.

Furthermore, during inflation of known blood pressure cuffs, multiple inner folds form in the bladder, which change in quantity, depth and shape during inflation and which deform the soft tissue of the upper arm. For instance, it is known from magnetic resonance images of conventional cylindrical inflated blood pressure cuffs that the folds are generally very pronounced, wherein they are more pronounced in the distal cuff region than in the proximal cuff region.

These folds, whose quantity, depth and shape change during inflation and deflation, lead to noise in the measured pressure pulses and thereby decrease the quality of these pressure pulses. In contrast, the blood pressure cuff described above forms less inner folds and forms these inner folds less pronounced, which also contributes to the increased quality of the measured pressure pulses.

In addition, the inner folds formed during inflation when using known blood pressure cuffs can, on a long term, hurt the skin and subcutaneous tissue and lead to bruises. Also, this can be avoided or reduced by using the blood pressure cuff described above because of forming less inner folds.

The blood pressure cuff, especially the shell, can be opened and closed and it can be easily applied to the upper arm of the subject for a self-measurement, i.e. for a measurement carried out by the subject himself/herself, and it can also be easily applied to the upper arm of the subject by a second person. For instance, the blood pressure cuff can be widened such that an inner circumference of the blood pressure cuff is larger than that of a closed hand and an elbow of the subject. The blood pressure cuff with this relatively large circumference and hence diameter then can be slid up over and along the closed hand, the lower arm and the elbow until it has reached the upper arm. When the blood pressure cuff has reached the upper arm, the circumference of the blood pressure cuff can be adapted to the circumference of the upper arm for allowing for a high-quality measurement of the pressure pulses. The blood pressure cuff can also be arranged around the upper arm by opening the blood pressure cuff above the mid of the upper arm and sliding it from the side onto the upper arm. In any case, the blood pressure cuff is tightened on the upper arm by using the flap 24 and aligned with the conical contour of the upper arm. A too tight or too loose attachment can be avoided and an optimal fit to the upper arm can be ensured much easier than with a known conventional cylindrical blood pressure cuff. This does not only contribute further to the high-quality measurement of the pressure pulses, which are used for determining the blood pressure, but it also avoids impressions on the upper arm.

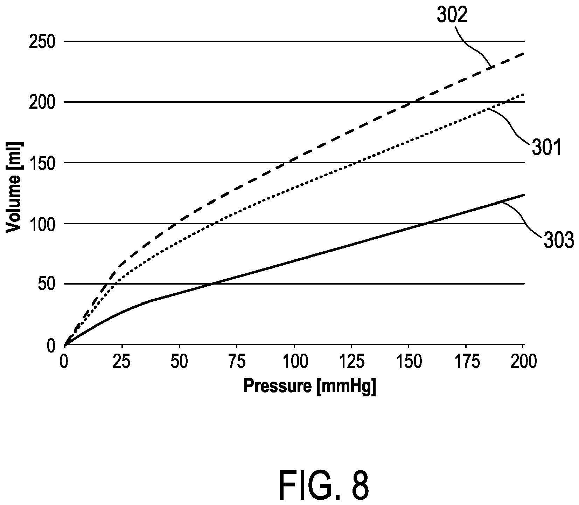

The increase of bladder volume with increasing applied pressure is smaller for the blood pressure cuff described above in comparison to the increase of bladder volume with increasing applied pressure for known blood pressure cuffs. This leads to the effect that, at a same applied pressure value, the bladder volume of the blood pressure cuff described above is smaller than the bladder volume of a known blood pressure cuff. This is illustrated in Fig. 8.

Thus, Fig. 8 schematically and exemplarily shows the bladder volume depending on the applied pressure, wherein line 301 indicates the volume of the bladder for the known blood pressure cuff Omron NIBP Intelli Wrap Cuff HEM-FL31, line 302 indicates the volume of the bladder for the known blood pressure cuff Philips Gentle Care Disposable Cuff and line 303 indicates the volume of the bladder for the blood pressure cuff described above, i.e. described with reference to Figs. 2 to 6. Fig. 8 shows very well that the bladder volume is significantly decreased, which also contributes to the improved quality of the measured pressure pulses that are used for determining the blood pressure. In particular, the bladder preferentially is covered completely by the shell avoiding any pulsation loss to the atmosphere.

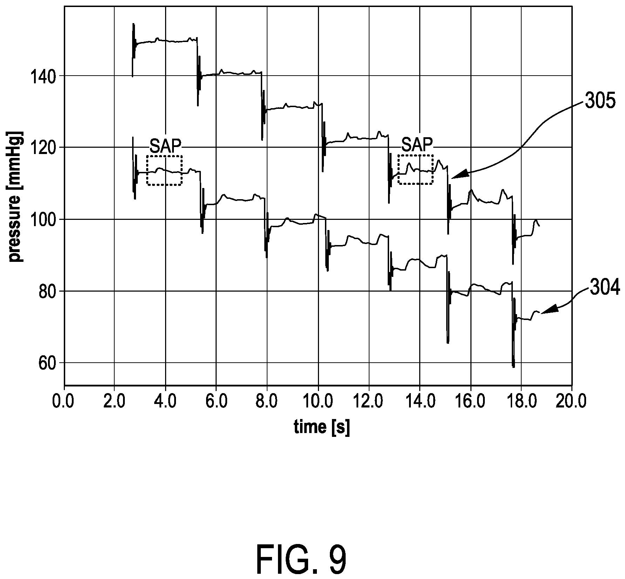

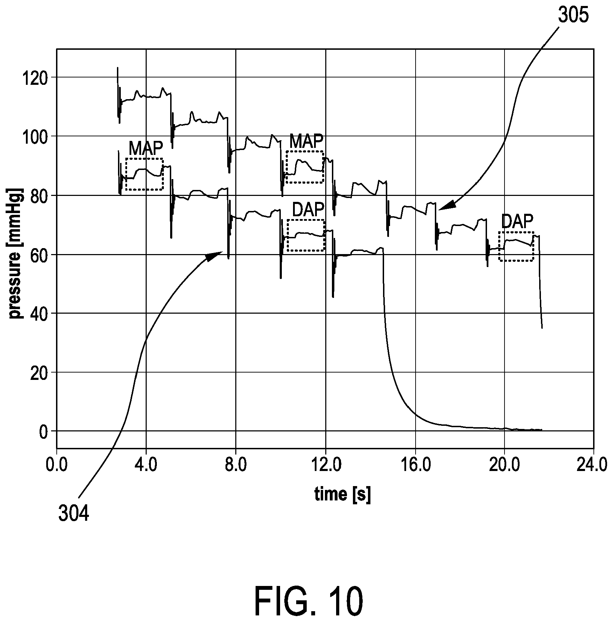

Figs. 9 and 10 schematically and exemplarily show measured pressure pulses, wherein reference number 304 indicates the pressure pulses measured by using the known blood pressure cuff Omron NIBP Intelli Wrap Cuff HEM-FL31 and reference number 305 indicates the pressure pulses measured by using the blood pressure cuff described above. As can be seen, if the blood pressure cuff as described above, i.e. as described above with reference to Figs. 2 to 6, is used, the amplitudes of the measured pressure pulses are much larger and also the contour features like the dicrotic notch are much better visible. There is also less noise.

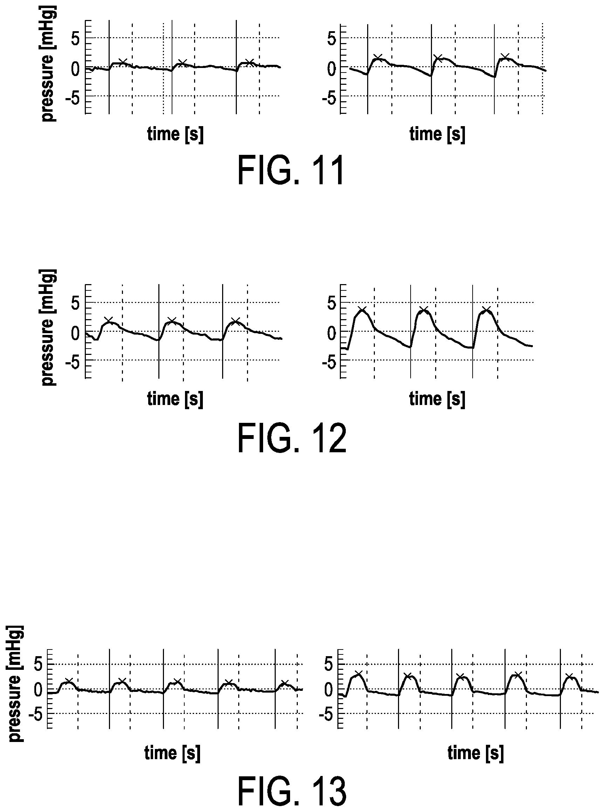

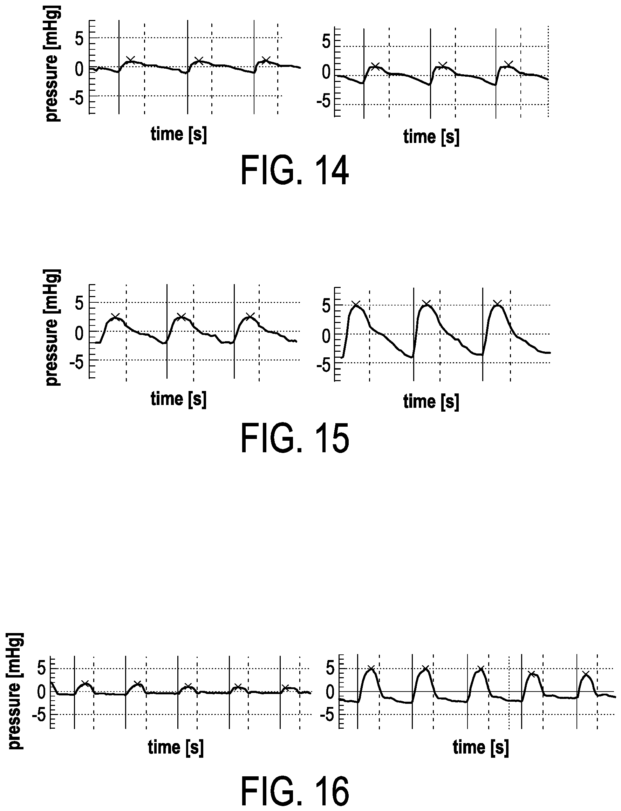

Figs. 11 to 16 compare measured pressure pulses, i.e. the pulsation signals, of the known blood pressure cuffs with the blood pressure cuff described above with reference to Figs. 2 to 6. Figs. 11, 12 and 13 show on the left side the pressure signals that have been measured by using the known blood pressure cuff Philips Gentle Care Disposable Cuff and on the right side the pressure signals that have been measured by using the blood pressure cuff described above, wherein Fig. 11 shows the pressure pulses at DAP, Fig. 12 shows the pressure pulses at MAP and Fig. 13 shows the pressure pulses at SAP. Figs. 14, 15 and 16 show on the left side the pressure signals that have been measured by using the known blood pressure cuff Omron NIBP Intelli Wrap Cuff HEM-FL31 and on the right side the pressure signals that have been measured by using the blood pressure cuff described above, wherein Fig. 14 shows the pressure pulses at DAP, Fig. 15 shows the pressure pulses at MAP and Fig. 16 shows the pressure pulses at SAP. As can also be seen in Figs. 11 to 16, the amplitude of the pressure pulses is larger and the pulse contour features are better visible if the blood pressure cuff described above with reference to Figs. 2 to 6 is used. Also here the pulse signal is less noisy.

As explained above, the outer layer of the bladder, i.e. a corresponding foil of the bladder, can be firmly attached, particularly glued or welded, to the inner side of the shell, wherein any air is avoided in between the outer layer of the bladder and the inner side of the shell. Thus, two materials can be used which are merged, wherein a first material is the material of the outer layer of the bladder and a second material is the material of the shell. The shell material is preferentially a HDPE/PP/ABS blend or a material with similar physical properties. The shell material preferentially is biocompatible and preferentially either biodegradable and/or recyclable. The outer layer of the bladder preferentially has a sleek outer side for facilitating airless merging, especially airless gluing, or welding, and preferentially is made of ripstop nylon, ripstop polyester or a combination of ripstop nylon and ripstop polyester. The inner layer of the bladder preferentially is thinner and/or shorter than the outer layer of the bladder. The inner layer of the bladder preferentially comprises skin-friendly material which is non-adhesive on the skin, and which optionally also easily glides on the skin especially under pressure. The inner layer material can be, for instance, TPU. Preferentially, all materials of the blood pressure cuff are biocompatible, and they can be biodegradable or recyclable.

The outer shell, which preferentially is larger than the main part of the bladder, which is inflatable, and which therefore completely covers this main part of the bladder, contributes to the avoidance of a pulse signal loss into the atmosphere as it happens with known conventional blood pressure cuffs because of ballooning. This in turn contributes to a largely improved maximal tissue pressure amplitude (TPPmx) and a largely improved maximal pulsation transfer (TFmx) from the enclosed artery or the enclosed arteries to the skin, wherein TFmx = TPPmx/PP with PP being the pulse pressure calculated from SAP - DAP. It has been found that, by using the blood pressure cuff described above, TPPmx could be doubled in comparison to the above-mentioned Philips blood pressure cuff and by more than 100% in comparison to the above-mentioned Omron blood pressure cuff. Moreover, the TFmx could be increased by more than 100% in comparison to the Philips blood pressure cuff and by 120% in comparison to the Omron blood pressure cuff. In addition, pulse contour details like the dicrotic notch and pulse waveform changes during inflation are much better visible in comparison to using the known Philips and Omron blood pressure cuffs as can also be seen in Figs. 9 to 16. The significantly higher TPPmx and TFmx enable a more accurate blood pressure value determination, particularly in severely obese subjects and those with stiff arteries in which blood pressure measurements with known conventional blood pressure cuffs often lack accuracy due to too low signal to noise ratio.

The lower bladder volume, which is explained above with reference to Fig. 8, reduces the dampening influence of air and thereby also allows for higher amplitude pulsations like a higher TPPmx. Moreover, the blood pressure cuff has, as described above, less skin and subcutaneous tissue damaging inner folds during bladder inflation and deflation and the blood pressure cuff can be applied to the subject for a self-measurement or for a measurement by a second person with a better alignment to the contour of the upper arm.

The shell preferentially is a kinking-proof shell. The kinking-proof shell exhibits a stiffness notably larger than the stiffness of the inner layer of the bladder and also of the outer layer of the bladder, with which the inner side of the shell is merged. The kinking-proof shell is a stiffening component which is configured for ensuring stiffness of the blood pressure cuff for pressure measurements. The kinking-proof shell may be the sole stiffening component of the blood pressure cuff, the stiffening component exhibiting a structural solidity or strength and being configured for providing stiffness against compression forces during pressure measurements.

Preferably, the kinking-proof shell is a stiffening one-piece shell. Such a design allows for providing sufficient stiffness in a straightforward manner.

Preferably, the kinking-proof shell is dimensioned so as to overlap when surrounding the body part of the subject. In other words, the kinking-proof shell preferably completely surrounds the subject's body part.

In an example the shell, particularly the kinking-proof shell, is made from metal and/or plastic, in particular fiber-reinforced plastic. For example, the shell might be made from or coated with a thermoplastic material, preferably made from polyurethane or a polyolefin, more preferably made from polyethylene or polyoxymethylene (POM). For example, the shell might be made from a thermoplastic material which provides a surface on which adhesives may adhere durably. If the shell comprises fiber-reinforced plastic material, the fibers may be natural fibers, organic fibers or inorganic fibers. In an embodiment, as explained above, the shell is made of a HDPE, PP, ABS blend.

In particular, if the shell is made from plastic material, in an example the shell exhibits a thickness within a range from 0.1 mm to 6 mm, more preferably within a range from 1 mm to 3 mm, even more preferably within range from 1.0 mm to 2.0 mm. For example, the shell may have a thickness of 1.5 mm, especially for adults. Or the shell may have a thickness of 0.1 mm to 0.5 mm, especially for premature babies, babies, and infants. In a preferred embodiment the shell is made from polyethylene (PE) having a thickness of 1.5 mm.

In an example, in order to avoid kinks on the one hand and to allow a reduction of the inner diameter on the other hand, the kinking-proof shell exhibits a modulus of elasticity of more than 50 MPa, more preferably within a range from 100 MPa to 10 GPa, even more preferably within a range from 200 MPa to 1 GPa.

Although in above-described embodiments the blood pressure cuff is wrapped around the upper arm, in other embodiments it can also be wrapped around another body part of the subject, especially around another extremity, e.g. the forearm.

Although in the above-described embodiment, in which the outer layer of the bladder is merged with the inner side of the shell, the inner layer of the bladder and the outer layer of the bladder are made of different materials, the inner layer of the bladder and the outer layer of the bladder can also be made of the same material.

Although in the above-described embodiment the outer layer of the bladder is merged with the inner side of the shell, it is also possible that the outer layer of the bladder is not merged with an inner side of a shell, but that the outer layer of the bladder is more rigid than the inner layer of the bladder, in order to fulfill the function of the shell, wherein in this case the blood pressure cuff might not even comprise any shell. In particular, the outer layer of the bladder can comprise a material being thicker and/or more rigid and/or harder than the material of the inner layer of the bladder. The outer layer of the bladder can comprise a reinforcing structure.

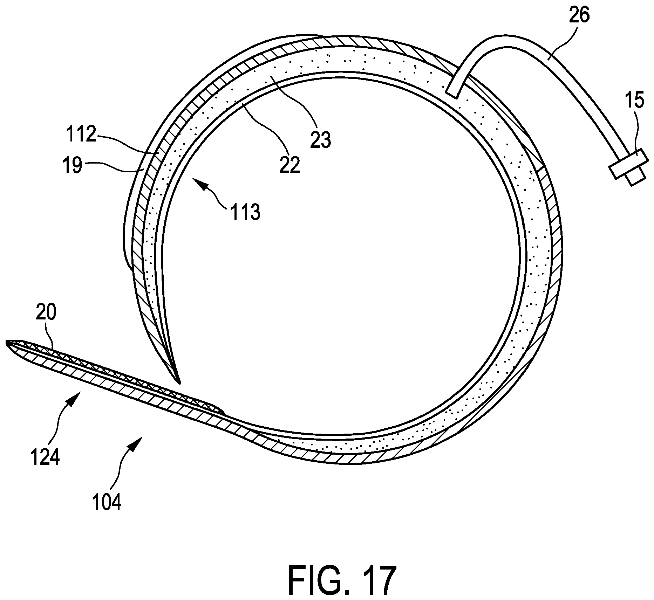

Fig. 17 schematically and exemplarily illustrates a corresponding further embodiment of the blood pressure cuff. In this embodiment, the blood pressure cuff 104 also comprises an inflatable bladder 113. As can be seen in Fig. 17, the bladder 113 comprises an outer layer 112 and an inner layer 22, wherein these two layers 112 and 22 are attached to each other, for instance, glued or welded, along their edges, in order to form in between these two layers 112, 22 a space 23 that can be filled with a fluid like air by using the tube 26 which comprises a connector 15 for connecting the tube 26 to the tube 14. However, in this embodiment the inner layer 22 of the bladder 113 is substantially less rigid than the outer layer 112. The inner layer 22 is non-adhesive on the subject's skin and preferentially TPU also in this embodiment. The outer layer 112 of the bladder 113 can be made of ripstop nylon, ripstop polyester or a combination of ripstop nylon and ripstop polyester. In a preferred embodiment the ripstop distance, i.e. the distance between the reinforcing yarns, is equal to or smaller than 5 mm.

Thus, also in the embodiment illustrated with reference to Fig. 17 the inner layer of the bladder can be thinner and shorter than the outer layer of the bladder and the inner layer of the bladder can be made of TPU or another material which is non-adhesive on the skin. In particular, also in this embodiment the inner layer can be made of a material that is skin-protective.

As mentioned above, if the outer layer of the bladder is merged with the shell, for instance, by gluing or welding, preferentially the shell is larger than the active bladder area, completely covers the active bladder and completely encloses the upper arm especially at any appropriate circumference, especially at maximum allowable circumference. In particular, the inner layer at the active part of the bladder preferentially is shorter than the shell and it preferentially has a minimum length of 80% of a maximum arm circumference. If in another embodiment the outer layer of the bladder is more rigid than the inner layer of the bladder and not merged with a shell, the outer layer can also be larger than the active bladder area, completely cover the active bladder and completely enclose the upper arm especially at any appropriate circumference and especially at maximum allowable circumference. In particular, the inner layer at the active part of the bladder can be shorter than the outer layer of the bladder and it can have a minimum length of 80% of a maximum arm circumference.

The active bladder area or the active part of the bladder preferentially refers to the area or part, respectively, of the bladder which exerts force on the surrounded body part. For instance, it can be defined by the parts of the bladder in which the fluid is flowable.

If the outer layer of the bladder is merged with the shell, for instance, by gluing or welding, the bladder also comprises the flap preferentially with the loop part of the hook and loop mechanism as explained above with reference to, for example, Fig. 4. The hook part is formed on the outer side of the shell as explained above, for instance, with respect to any of Figs. 3, 5 and 6. If in another embodiment the outer layer of the bladder is more rigid than the inner layer of the bladder and not merged with a shell, both, the hook part and the loop part, can be part of a bladder extension.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Determinations like the determination of the blood pressure depending on the measured pressure pulses and/or the control of the blood pressure determination system such that a) the bladder of the blood pressure cuff is inflated and deflated, b) the pressure pulses are measured during inflation and/or deflation and c) the blood pressure is determined based on the measured pressure pulses, can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a blood pressure cuff configured to surround a body part of a subject for determining the blood pressure of the subject, wherein the blood pressure cuff comprises an inflatable bladder with an inner layer and an outer layer, wherein a) the blood pressure cuff further comprises a shell having an inner side merged with the outer layer of the bladder, and/or b) the outer layer of the bladder is more rigid than the inner layer of the bladder. By using such a blood pressure cuff, the quality of blood pressure measurements can be improved.

## Claims

1. A blood pressure cuff configured to surround a body part (11) of a subject (2) for determining the blood pressure of the subject (2), wherein the blood pressure cuff (4; 104) comprises an inflatable bladder (13; 113) with an inner layer (22) and an outer layer (16; 112), wherein a) the blood pressure cuff (4; 104) further comprises a shell (12) having an inner side (17) merged with the outer layer (16) of the bladder, and/or b) the outer layer (112) of the bladder is more rigid than the inner layer (22) of the bladder.

2. The blood pressure cuff of claim 1, wherein the outer layer (16) of the bladder (13) and the inner side (17) of the shell (12) are merged by gluing or welding them together.

3. The blood pressure cuff of any of claims 1 and 2, wherein the shell (12) is conical.

4. The blood pressure cuff of any of the preceding claims, wherein the shell (12) comprises a high-density polyethylene (HDPE), polypropylene (PP) and acrylonitrile butadiene styrene (ABS) blend.

5. The blood pressure cuff of any of the preceding claims, wherein the inner layer (22) of the bladder (113) and the outer layer (112) of the bladder (113) comprise different materials.

6. The blood pressure cuff of any of the preceding claims, wherein the outer layer (112) of the bladder (113) comprises a material being thicker, more rigid and/or harder than the material comprised by the inner layer (22) of the bladder (113).

7. The blood pressure cuff of any of the preceding claims, wherein the outer layer (112) of the bladder (113) comprises a reinforcing structure.

8. The blood pressure cuff of any of the preceding claims, wherein the outer layer (16; 112) of the bladder (13; 113) comprises a ripstop material.

9. The blood pressure cuff of any of the preceding claims, wherein the inner layer (22) of the bladder (13; 113) comprises at least one of thermoplastic polyurethane (TPU) and phthalate-free polyvinyl chloride (PVC).

10. The blood pressure cuff of any of the preceding claims, wherein a ratio of the area of the outer layer (16; 112) of the bladder (13; 113) relative to the area of the inner layer (22) of the bladder (13; 113) is larger than one.

11. The blood pressure cuff of any of the preceding claims, wherein the blood pressure cuff (4; 104) further comprises a flap (24; 124) with an attachment means (19, 20) for attaching the flap (24; 124) to the outer side of the blood pressure cuff (4; 104), in order to thereby fasten the blood pressure cuff (4; 104) when it surrounds the subject's body part (11), wherein the flap (24; 124) comprises at least one of the outer layer (16; 112) and the inner layer (22) of the bladder (13; 113).

12. A set of blood pressure cuffs of any of claim 1 to 11, wherein the set includes blood pressure cuffs (4; 104) with shells (12; 112) having different cone angles.

13. Use of the set of blood pressure cuffs defined by claim 12 for determining the blood pressure of subjects (2) having different subject lengths, wherein the subject length is a length of a predefined part of the body of the subject or a length of the entire body of the subject, wherein the set comprises a first blood pressure cuff (4; 104) for subjects (2) having a subject length being larger than a predefined length threshold and a second blood pressure cuff (4; 104) for subjects (2) having a subject length being smaller than the predefined length threshold, wherein the cone angle of the first blood pressure cuff (4; 104) is smaller than the cone angle of the second blood pressure cuff (4; 104), wherein, for determining the blood pressure for subjects (2) having a subject length being larger than the predefined length threshold, the first blood pressure cuff (4; 104) is used and, for determining the blood pressure for subjects (2) having a subject length being smaller than the predefined length threshold, the second blood pressure cuff (4; 104) is used.

14. A blood pressure determination system for determining the blood pressure of a subject (2), the blood pressure determination system (1) comprising:
- a blood pressure cuff (4; 104) as defined by any of claims 1 to 11,
- an inflation device (5) configured to inflate and deflate the bladder (13; 113) of the blood pressure cuff (4; 104),
- a pressure measuring device (6) being configured to measure the pressure in the bladder (13; 113) during inflation and/or deflation and
- a blood pressure determination device (7) for determining the blood pressure based on the measured pressure in the bladder (13; 113).

15. A blood pressure determination method comprising:
- wrapping a blood pressure cuff (4; 104) as defined by any of claims 1 to 11 around a body part (11) of a subject (2),
- inflating and deflating the bladder (13; 113) of the blood pressure cuff (4; 104) by an inflation device (5),
- measuring the pressure in the bladder (13; 113) by a pressure measuring device (6) during inflation and/or deflation,
- determining the blood pressure based on the measured pressure in the bladder (13; 113) by a blood pressure determination device (7).
